(19) Europäisches Patentamt
European Patent Office
Office européen des brevets

(11) **EP 3 919 891 A1**

(12) **EUROPEAN PATENT APPLICATION**
published in accordance with Art. 153(4) EPC

(43) Date of publication:
**08.12.2021 Bulletin 2021/49**

(51) Int Cl.:
*G01N 21/3577* (2014.01)    *G01N 21/552* (2014.01)
*A61B 5/1455* (2006.01)

(21) Application number: **20747822.3**

(22) Date of filing: **10.01.2020**

(86) International application number:
**PCT/JP2020/000677**

(87) International publication number:
**WO 2020/158348 (06.08.2020 Gazette 2020/32)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.01.2019   JP 2019016222**

(71) Applicants:
• **Tohoku University**
  **Sendai-shi, Miyagi 980-8577 (JP)**

• **SUMITOMO ELECTRIC INDUSTRIES, LTD.**
  **Osaka-shi, Osaka 541-0041 (JP)**

(72) Inventors:
• **MATSUURA, Yuji**
  **Sendai-shi, Miyagi 980-8577 (JP)**
• **HASHIMOTO, Jun-ichi**
  **Osaka-shi, Osaka 541-0041 (JP)**

(74) Representative: **Grünecker Patent- und
Rechtsanwälte
PartG mbB
Leopoldstraße 4
80802 München (DE)**

(54) **DEVICE AND METHOD FOR MEASURING BLOOD SUGAR LEVEL**

(57)    A blood-sugar-level measuring apparatus is a blood-sugar-level measuring apparatus using infrared spectroscopy. The blood-sugar-level measuring apparatus includes a light source that emits a laser light in an infrared region, a lens that converges the laser light, an optical fiber in which the laser light converged by the lens enters, and a prism in which the laser light emitted from the optical fiber enters.

FIG. 1A

FIG. 1B

FIG. 1C

EP 3 919 891 A1

**Description**

Technical Field

The present disclosure relates to a blood-sugar-level measuring apparatus and a blood-sugar-level measuring method

The present application claims the priority based on Japanese Patent Application No. 2019-016222 filed on January 31, 2019, the entire contents of which are incorporated herein by reference.

Background Art

There are several known noninvasive blood-sugar-level measurement methods using infrared spectroscopy (e.g., Patent Literatures 1 and 2). In Patent Literature 1, infrared light with a wavelength of 1020 cm$^{-1}$ to 1040 cm$^{-1}$ is used, and in Patent Literature 2, near-infrared light is used.

Citation List

Patent Literature

PTL 1: Domestic Re-publication of PCT International Publication No. 2006/011487
PTL 2: Japanese Unexamined Patent Application Publication No. 2014-18478

Summary of Invention

A blood-sugar-level measuring apparatus according to the present disclosure is a blood-sugar-level measuring apparatus using infrared spectroscopy. The blood-sugar-level measuring apparatus includes a light source configured to emit a laser light in an infrared region, a lens configured to converge the laser light, an optical fiber in which the laser light converged by the lens enters, and a prism in which the laser light emitted from the optical fiber enters.

A blood-sugar-level measuring apparatus according to the present disclosure is a blood-sugar-level measuring apparatus using infrared spectroscopy. The blood-sugar-level measuring apparatus includes a light source configured to emit a laser light in an infrared region, a lens configured to converge the laser light, and a prism in which the laser light converged by the lens enters.

A blood-sugar-level measuring apparatus according to the present disclosure is a blood-sugar-level measuring apparatus using infrared spectroscopy. The blood-sugar-level measuring apparatus includes a light source configured to emit a laser light in an infrared region and a prism in which the laser light enters. The prism has a surface by which the laser light is reflected, and a lower limit of an incident angle of the laser light with respect to the surface is a critical angle plus 3°.

A blood-sugar-level measuring apparatus according to the present disclosure is a blood-sugar-level measuring apparatus using infrared spectroscopy. The blood-sugar-level measuring apparatus includes a light source configured to emit a laser light in an infrared region, an optical fiber in which the laser light enters, and configured to have a tapered shape tapered at a tip portion thereof, and a prism in which the laser light emitted from the tip portion enters.

A blood-sugar-level measuring method according to the present disclosure is a method for measuring a blood-sugar-level using infrared spectroscopy. The method includes bringing a prism into contact with a subject, causing a laser light in an infrared region to enter the prism through a converging lens and an optical fiber, and detecting the laser light emitted from the prism.

A blood-sugar-level measuring method according to the present disclosure is a method for measuring a blood-sugar-level using infrared spectroscopy. The method includes bringing a prism into contact with a subject, causing a laser light in an infrared region to enter the prism through a converging lens, and detecting the laser light emitted from the prism.

A blood-sugar-level measuring method according to the present disclosure is a method for measuring a blood-sugar-level using infrared spectroscopy. The method includes bringing a prism into contact with a subject, causing a laser light in an infrared region to enter the prism, and detecting the laser light emitted from the prism. The prism has a surface by which the laser light is reflected, and a lower limit of an incident angle of the laser light with respect to the surface is a critical angle plus 3°.

A blood-sugar-level measuring method according to the present disclosure is a method for measuring a blood-sugar-level using infrared spectroscopy. The method includes bringing a prism into contact with a subject, causing a laser light in an infrared region to enter the prism through an optical fiber, and detecting the laser light emitted from the prism. The optical fiber has a tapered shape tapered at a tip portion thereof.

Brief Description of Drawings

[Fig. 1A] Fig. 1A is a diagram schematically illustrating a blood-sugar-level measuring apparatus according to an Embodiment 1.
[Fig. 1B] Fig. 1B is an enlarged view of an area around a lens and a prism.
[Fig. 1C] Fig. 1C is an enlarged view of the prism.
[Fig. 2A] Fig. 2A is a diagram illustrating an experiment.
[Fig. 2B] Fig. 2B is a diagram illustrating an intensity of light.
[Fig. 2C] Fig. 2C is a diagram illustrating a relationship between half width and distance d.
[Fig. 3A] Fig. 3A is a diagram illustrating a light source in a modification 1 of Embodiment 1.
[Fig. 3B] Fig. 3B is a diagram illustrating a blood-sugar-level measuring apparatus according to a modification 2 of Embodiment 1.
[Fig. 4A] Fig. 4A is a diagram schematically illustrating a blood-sugar-level measuring apparatus according to an Embodiment 2.
[Fig. 4B] Fig. 4B is an enlarged view of the prism.
[Fig. 5] Fig. 5 is an enlarged view of an optical fiber and a prism in an Embodiment 3.
[Fig. 6A] Fig. 6A illustrates a sample used in the experiment.
[Fig. 6B] Fig. 6B is a diagram illustrating measurement results of loss.

Description of Embodiments

[Problems to be solved by Present Disclosure]

A Fourier Transform Infrared Spectrometer (FTIR) is used as an apparatus for measuring a blood-sugar-level, but such a measuring apparatus is large and expensive.
By using a laser light source such as a quantum cascade laser (QCL), it is possible to make the apparatus smaller and less expensive. However, since the laser light is coherent light, the laser light interferes to locally have a higher intensity than light of the lamp. Therefore, it is easily affected by a distribution of a blood glucose in a living body, and the measurement accuracy is insufficient. Therefore, it is an object of the present disclosure to provide a blood-sugar-level measuring apparatus and a blood-sugar-level measuring method that can improve the measurement accuracy.

[Advantageous effects of Present Disclosure]

According to the present disclosure, it is possible to improve the measurement accuracy.

[Description of Embodiments of Present Disclosure]

First, embodiments of the present disclosure will be listed and described.
An aspect of the present disclosure is (1) a blood-sugar-level measuring apparatus using infrared spectroscopy. The blood-sugar-level measuring apparatus includes a light source configured to emit a laser light in an infrared region, a lens configured to converge the laser light, an optical fiber in which the laser light converged by the lens enters, and a prism in which the laser light emitted from the optical fiber enters. Since the laser light that is spread more can be incident on the prism as compared with the case without passing through the lens, the measurement accuracy is improved.
(2) A blood-sugar-level measuring apparatus is a blood-sugar-level measuring apparatus using infrared spectroscopy. The blood-sugar-level measuring apparatus includes a light source configured to emit a laser light in an infrared region, a lens configured to converge the laser light, and a prism in which the laser light converged by the lens enters. Since the laser light that is spread more can be incident on the prism as compared with the case without passing through the lens, the measurement accuracy is improved.
(3) A focal length of the lens may be greater than or equal to 2 mm and less than or equal to 20 mm. This allows the laser light to be spread out more, which improves the measurement accuracy.
(4) A blood-sugar-level measuring apparatus is a blood-sugar-level measuring apparatus using infrared spectroscopy. The blood-sugar-level measuring apparatus includes a light source configured to emit a laser light in an infrared region and a prism in which the laser light enters. The prism has a surface by which the laser light is reflected, and a lower limit of an incident angle of the laser light with respect to the surface is a critical angle plus 3°. This increases the number of reflections and the penetration depth of the light in the prism and suppresses interference, thus improving the measurement accuracy.

(5) A blood-sugar-level measuring apparatus is a blood-sugar-level measuring apparatus using infrared spectroscopy. The blood-sugar-level measuring apparatus includes a light source configured to emit a laser light in an infrared region, an optical fiber in which the laser light enters and that has a tapered shape tapered at a tip portion thereof, and a prism in which the laser light emitted from the tip portion enters. Since the laser light spreading from an optical fiber having a tapered shape can be incident on the prism, the measurement accuracy is improved.

(6) The light source may be a quantum cascade laser. This makes the blood-sugar-level measuring apparatus low cost and compact.

(7) A blood-sugar-level measuring method is a method for measuring a blood-sugar-level using infrared spectroscopy. The method includes bringing a prism into contact with a subject, causing a laser light in an infrared region to enter the prism through a converging lens and an optical fiber, and detecting the laser light emitted from the prism.

Since the laser light that is spread more can be incident on the prism compared with the case without passing through the converging lens, the measurement accuracy is improved.

(8) A blood-sugar-level measuring method is a method for measuring a blood-sugar-level using infrared spectroscopy. The method includes bringing a prism into contact with a subject, causing a laser light in an infrared region to enter the prism through a converging lens, and detecting the laser light emitted from the prism. Since the laser light that is spread more can be incident on the prism compared with the case without passing through the converging lens, the measurement accuracy is improved.

(9) A blood-sugar-level measuring method according to the present disclosure is a method for measuring a blood-sugar-level using infrared spectroscopy. The method includes bringing a prism into contact with a subject, causing a laser light in an infrared region to enter the prism, and detecting the laser light emitted from the prism. The prism has a surface by which the laser light is reflected, and a lower limit of an incident angle of the laser light with respect to the surface is a critical angle plus 3°. This increases the number of reflections and the penetration depth of the light in the prism and suppresses interference, thus improving the measurement accuracy.

(10) A blood-sugar-level measuring method is a method for measuring a blood-sugar-level using infrared spectroscopy. The method includes bringing a prism into contact with a subject, causing a laser light in an infrared region to enter the prism through an optical fiber, and of detecting the laser light emitted from the prism. The optical fiber has a tapered shape tapered at a tip portion thereof. Since the laser light spreading from the optical fiber having the tapered shape can be incident on the prism, the measurement accuracy is improved.

[Details of Embodiments of Present Disclosure]

Specific examples of a blood-sugar-level measuring apparatus and a blood-sugar-level measuring method according to an embodiment of the present disclosure will be described below with reference to the drawings. It should be noted that the present disclosure is not limited to these examples, and is defined by Claims, and is intended to embrace all the modifications within the meaning and range of equivalency of the Claims.

Embodiment 1

(Blood-sugar-level measuring apparatus)

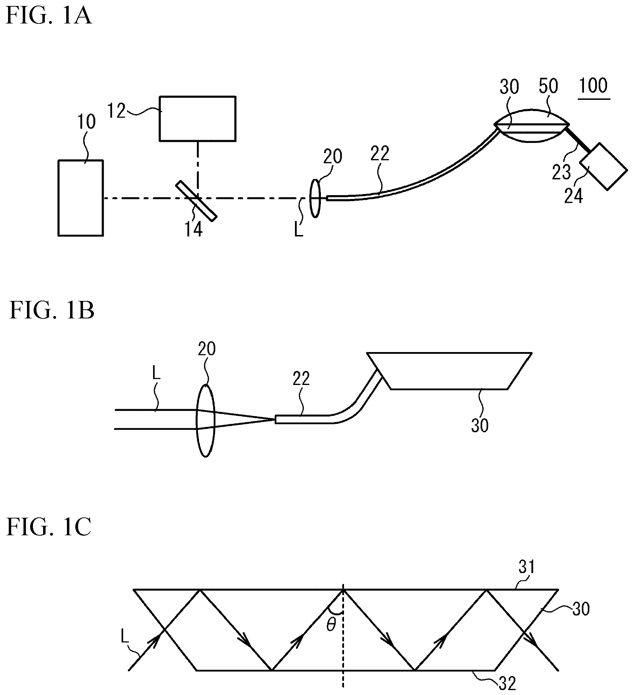

Fig. 1A is a diagram illustrating a blood-sugar-level measuring apparatus 100 according to Embodiment 1. Fig. 1B is an enlarged view of an area around a lens 20 and a prism 30. As illustrated in Fig. 1A, blood-sugar-level measuring apparatus 100 includes QCLs 10 and 12, a mirror 14, lens 20, optical fibers 22 and 23, a detector 24, and prism 30. The blood-sugar-level measuring apparatus 100 is an apparatus for measuring a blood-sugar-level by infrared spectroscopy, more specifically, an infrared attenuated total reflection method (ATR method).

QCLs 10 and 12 are laser light sources that oscillate at a wavelength (from 4 $\mu$m to 10 $\mu$m) in a mid-infrared region, for example. The wavelengths of the lights emitted from QCLs 10 and 12 are different from each other. Both of the wavelengths of the lights emitted from QCLs 10 and 12 may be within a predetermined range such as $\pm$10 cm$^{-1}$ centered on a wavelength of an absorption peak of glucose. Further, one may be within the predetermined range centered on the wavelength of the absorption peak, and the other may be outside the range centered on the absorption peak. The absorption peaks are located, for example, around 1155 cm$^{-1}$, around 1080 cm$^{-1}$, and around 1035 cm$^{-1}$. Lens 20 is a converging lens and converges the laser light emitted from QCLs 10 and 12. A focal length is 8 mm, for example.

One end of optical fiber 22 faces lens 20, and the other end is attached to one of the side surfaces of prism 30, for example, closely attached to one of the side surfaces of prism 30. One end of optical fiber 23 is attached to the other side surface of prism 30, and the other end of optical fiber 23 is attached to detector 24. Optical fibers 22 and 23 are hollow fibers having diameters of 2 mm, for example, and can transmit infrared light with a low loss. Detector 24 is, for example, an indium arsenide antimonide (InAsSb) detector.

Prism 30 is, for example, a trapezoidal attenuated total reflection prism (ATR prism) formed of ZnS or the like. Two bottom surfaces 31 and 32 of prism 30 face each other. A thickness of prism 30, which is a distance between the bottom surfaces, is 2.4 mm, for example. A length of bottom surface 31 is 24 mm, for example, and a length of bottom surface 32 is smaller than that of bottom surface 31.

When laser light L incident on lens 20 is collimated, a length between one end of optical fiber 22 and lens 20 is preferably equal to the focal length of lens 20, for example, 8 mm.

Next, a measurement of a blood-sugar-level will be described. As illustrated in Fig. 1A, prism 30 is sandwiched by a lip 50 of a subject, and lip 50 is brought into contact with bottom surfaces 31 and 32. Laser lights are emitted from QCLs 10 and 12. The light emitted from QCL 10 is transmitted through mirror 14, and the light emitted from QCL 12 is reflected by mirror 14. As illustrated in Fig. 1B, these lights (laser light L) are converged by lens 20 to enter optical fiber 22, and then propagate through optical fiber 22 to enter prism 30.

Fig. 1C is an enlarged view of prism 30. As illustrated in Fig. 1C, laser light L is reflected by bottom surfaces 31 and 32 of prism 30. An evanescent field generated at a boundary between prism 30 and the outside of prism 30 upon reflection is absorbed by a sample (subject). More specifically, glucose or the like in the interstitial fluid of the subject absorbs infrared light. The light after absorption is emitted from prism 30, and propagates through optical fiber 23 illustrated in Fig. 1A to enter detector 24. The blood-sugar-level can be measured from a mid-infrared absorption spectrum obtained by this method.

Although five reflections occur in prism 30 in Fig. 1C, the number of reflections may be less than five, or five or more. In order to improve a sensitivity, it is preferable that reflection occurs five times or more, for example, nine times. An incident angle $\theta$ of laser light L with respect to bottom surfaces 31 and 32 of prism 30 is an angle formed by a normal line of the bottom surface of prism 30 and laser light L, and is preferably an angle equal to or greater than a critical angle, for example, 45°.

In Embodiment 1, as illustrated in Fig. 1B, laser light L is converged by lens 20 to enter optical fiber 22. As a result, laser light L is emitted from optical fiber 22 in a spread state compared with the case without transmitting the lens 20, and is incident on prism 30. Therefore, the measurement accuracy of the blood-sugar-level is improved. An experiment on a spread of light will be described below.

(Experiment)

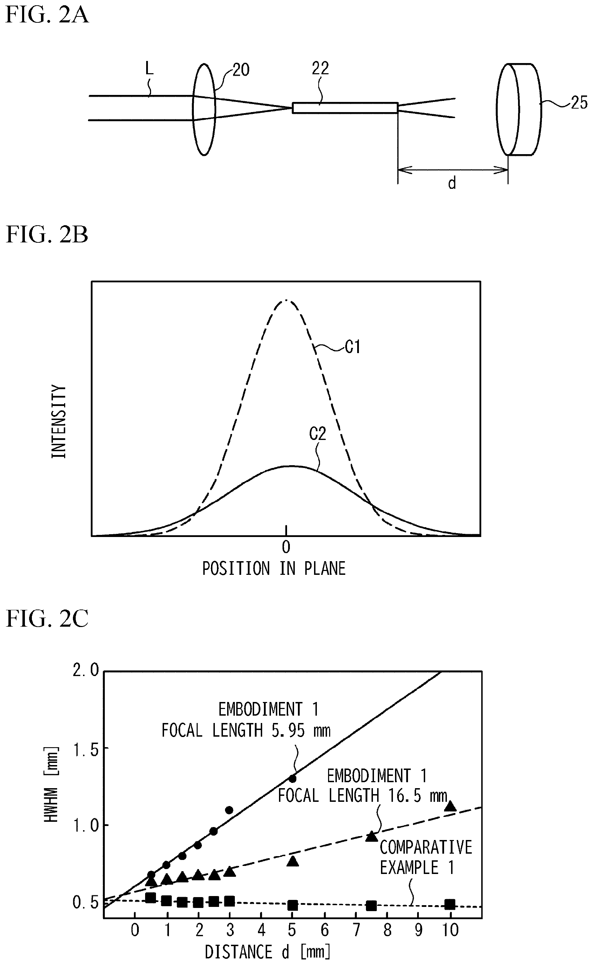

Fig. 2A illustrates an experiment. As illustrated in Fig. 2A, lens 20, optical fiber 22, and detector 25 were arranged in this order. In Fig. 2A, laser light L was incident on lens 20 from light sources (QCLs 10 and 12, not shown). Laser light L then propagated through optical fiber 22, and was incident on detector 25. Optical fiber 22 had a diameter of 2 mm and a length of 15 cm. Detector 25 was formed of indium arsenide antimonide (InAsSb), for example. In this experiment, the light intensity was detected while changing a distance d between optical fiber 22 and detector 25 in each of a comparative example 1 without lens 20, an example according to Embodiment 1 with lens 20 having a focal length of 16.5 mm, and an example according to Embodiment 1 with lens 20 having a focal length of 5.95 mm.

Fig. 2B is a diagram illustrating an intensity of light. A horizontal axis represents a position in the plane of detector 25, and a vertical axis represents the intensity of the emitted light. As illustrated in Fig. 2B, the intensity of light detected by detector 25 draws a Gaussian-type curve. On the surface of detector 25 facing optical fiber 22, the intensity has a peak at the position in front of optical fiber 22 (in-plane position is 0), and decreases as the in-plane position is shifted away from 0. As compared with a curve C1 indicated by a broken line, a curve C2 indicated by a solid line has a lower peak intensity and a larger half width at half maximum (HWHM). A large HWHM, like curve C2, indicates that light is spread and light having a high intensity is obtained in a wide area.

Fig. 2C illustrates a relationship between half-value width and distance d. A horizontal axis represents a distance d between optical fiber 22 and detector 25, and a vertical axis represents a HWHM A dotted line and squares represent the comparative example 1, a broken line and triangles represent the example in which the focal length is 16.5 mm, and a solid line and circles represent the example in which the focal length is 5.95 mm. In the comparative example 1 in which lens 20 is not used, the HWHM is smaller than those in the other examples, and an increase in the HWHM with an increase in the distance d is not detected.

On the other hand, the HWHM of Embodiment 1 with lens 20 is larger than that of the comparative example 1, and the HWHM also increases as the distance d increases. In particular, in the case where the focal length was 5.95 mm, the HWHM was larger than those in the other two cases. This indicates that the use of lens 20 having a small focal length spreads the light emitted from optical fiber 22. Specifically, when the focal length is 16.5 mm, a spread of the light from the end of optical fiber 22 is 2.8°, and when the focal length is 5.95 mm, the spread is 8.4°.

According to Embodiment 1, laser light L converged by lens 20 is incident on prism 30. Therefore, the laser light L in a spread state can be incident on the prism 30. As a result, high intensity can be obtained over a wide area, the influence of a blood sugar distribution can be suppressed, and the measurement accuracy can be improved.

As illustrated in Fig. 2C, shortening the focal length of lens 20 can make the spread of light larger. The focal length is preferably equal to or greater than 2 mm and equal to or less than 20 mm, for example.

As illustrated in Fig. 1A, the light sources of blood-sugar-level measuring apparatus 100 are QCLs 10 and 12. Therefore, compared with an apparatus using FTIR, the apparatus 100 is low in cost, small in size, and excellent in portability. Prism 30 is connected to optical fibers 22 and 23. Optical fibers 22 and 23 are preferably hollow fibers that are flexible and transmit infrared light. As a result, the blood-sugar-level can be measured with prism 30 sandwiched by the lip of the subject. Since the stratum corneum of the lip is thinner than the skin, mid-infrared light easily enters, and the detection sensitivity is improved. Blood-sugar-level measuring apparatus 100 may not include optical fibers 22 and 23.

Although two quantum cascade laser QCLs 10 and 12 are used as the light sources, three or more QCLs may be used, for example. For example, two of the wave numbers of the plurality of emitted lights may be located at the absorption peak of glucose, and one of the wave numbers may be located outside the absorption peak of glucose. The one of the wave numbers can be used as a background.

(Modification 1)

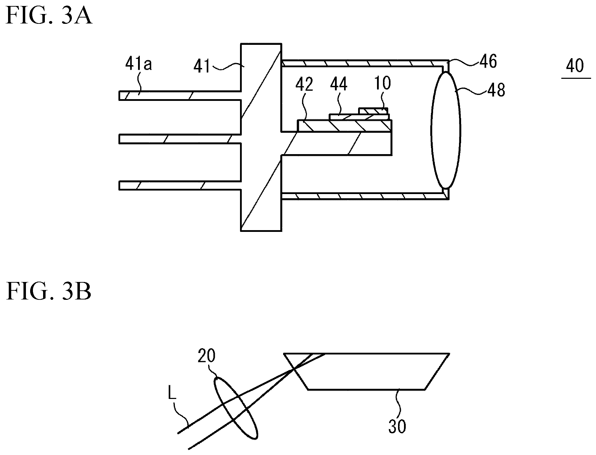

Fig. 3A is a diagram illustrating a light source according to Modification 1 of Embodiment 1. A CAN module 40 with a lens illustrated in Fig. 3A can be used instead of QCL 10 in Fig 1A. CAN module 40 includes a package 41, a thermoelectric cooler (TEC) 42, a submount 44, a cap 46, a lens 48, and QCL 10. TEC 42 is mounted in package 41. Submount 44 is mounted on TEC 42. QCL 10 is mounted on submount 44. By attaching cap 46 to package 41, the inside of package 41 is hermetically sealed. Lens 48 is provided at a portion of cap 46 facing QCL 10.

Lead pins 41a provided on the package 41 can be used to input and output electrical signals to QCL 10 and TEC 42. TEC 42 includes, for example, a Peltier element to perform temperature control. Lens 48 is a collimating or converging lens. Instead of QCL 12 in Fig. 1A, CAN module 40 having QCL 12 can be used. When lens 48 is the converging lens, the converged light is incident on optical fiber 22. Therefore, lens 20 illustrated in Fig. 1A may not be provided.

(Modification)

Fig. 3B is a diagram illustrating a blood-sugar-level measuring apparatus according to Modification 2 of Embodiment 1, and is an enlarged view of lens 20 and prism 30. As illustrated in Fig. 3B, laser light L converged by lens 20 is incident on prism 30 without passing through the optical fiber. The distance between lens 20 and the side surface facing lens 20 of prism 30 is preferably equal to or less than the focal length of lens 20, for example. According to Modification 2, similarly to Embodiment 1, laser light L in the spread state can be incident on prism 30. As a result, high intensity can be obtained over the wide area, the influence of the blood sugar distribution can be suppressed, and the measurement accuracy can be improved.

Embodiment 2

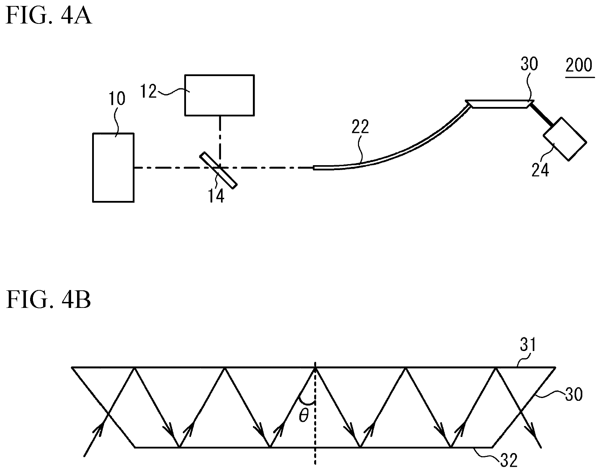

Fig. 4A is a diagram illustrating a blood-sugar-level measuring apparatus 200 according to Embodiment 2. As illustrated in Fig. 4A, blood-sugar-level measuring apparatus 200 does not have lens 20. A laser light L is incident on optical fiber 22 without passing through lens 20. Other configurations are the same as those in Embodiment 1. In addition, prism 30 is brought into contact with the lip or the like of the subject, laser light is incident from QCLs 10 and 12, and infrared light is detected by detector 24 to measure the blood-sugar-level in the same manner as in Embodiment 1.

Fig. 4B is an enlarged view of prism 30. The incident angle $\theta$ of laser light L with respect to bottom surfaces 31 and 32 will be described. The refractive index of prism 30 is n1, and the refractive index of a sample (biological tissue such as a human body) in contact with prism 30 is n2. The critical angle $\theta c$ at the boundary between prism 30 and the sample is given by the following equation.

$$\theta c = \sin^{-1} (n2/n1)$$

In the vicinity of the critical angle $\theta c$, an evanescent field rapidly increases, and absorption by the sample becomes too large, so that an S/N ratio may greatly decrease. Therefore, the lower limit of the incident angle $\theta$ with respect to bottom surfaces 31 and 32 is set to, for example, $\theta c + 3°$. For example, when prism 30 is ZnS, the refractive index n1 is 2.2. The refractive index n2 of the biological tissue to be measured is 1.2, and the critical angle $\theta c$ is 33°. Therefore, the lower limit of the incident angle $\theta$ is set to, for example, 36° which is $\theta c + 3°$, and the upper limit thereof is 45°, for example.

According to Embodiment 2, the lower limit of the incident angle is set to θc + 3°. This increases the number of reflections of laser light L and suppresses interference. Therefore, high intensity is obtained over the wide area, the influence of the blood sugar distribution is suppressed, and a highly accurate blood-sugar-level measurement is possible. In addition, since the penetration depth of the evanescent field is increased by making the laser light incident at an angle with respect to the critical angle, the intensity is increased and the measurement accuracy is improved. The lower limit of the incident angle may be smaller than θc + 3°, and also may be θc + 4° or θc + 5°, for example.

Embodiment 3

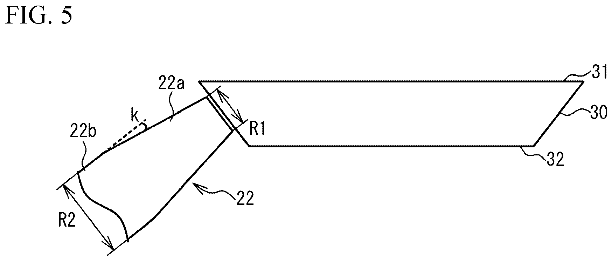

Fig. 5 is an enlarged view of an optical fiber 22 and a prism 30 according to Embodiment 3. As illustrated in Fig. 5, optical fiber 22 has a tip portion 22a and a straight portion 22b. Tip portion 22a is provided in a tip portion of straight portion 22b so as to face prism 30. Tip portion 22a has a tapered shape tapered toward prism 30. A diameter R1 of the end face of tip portion 22a of optical fiber 22 is 1. 6 mm, for example, and a diameter R2 of straight portion 22b is 2 mm, for example. A tilt angle k of tip portion 22a with respect to straight portion 22b is 5° or more and 15° or less, for example. Other configurations and the method for measuring the blood-sugar-level are the same as those in Embodiment 2.

According to Embodiment 3, since optical fiber 22 has tip portion 22a with the tapered shape, light emitted from tip portion 22a is spread and enters prism 30. Therefore, it is possible to suppress the influence of the blood sugar distribution, and the measurement accuracy can be improved.

Embodiment 4

Embodiment 4 is an embodiment in which Embodiment 1 and Embodiment 2 are combined. The configuration of a blood-sugar-level measuring apparatus is the same as in Fig. 1A. According to Embodiment 4, light converged by lens 20 is incident on prism 30 at the incident angle in Embodiment 2. Thus, the blood-sugar-level can be measured with high accuracy.

Embodiment 5

Embodiment 5 is an embodiment in which Embodiment 2 and Embodiment 3 are combined. The configuration of a blood-sugar-level measuring apparatus is the same as in Fig. 4A, and an optical fiber 22 has tip portion 22a illustrated in Fig. 5. According to Embodiment 5, light emitted from tip portion 22a of optical fiber 22 is incident on prism 30 at the incident angle in Embodiment 2. Thus, the blood-sugar-level can be measured with high accuracy.

(Experiment)

In examples according to Embodiments 1 to 5 and a comparative example 2, a loss of incident light on prism 30 was measured. The comparative example 2 was an example in which lens 20 and optical fiber 22 having a tapered shape were not used and the incident angle was 45°.

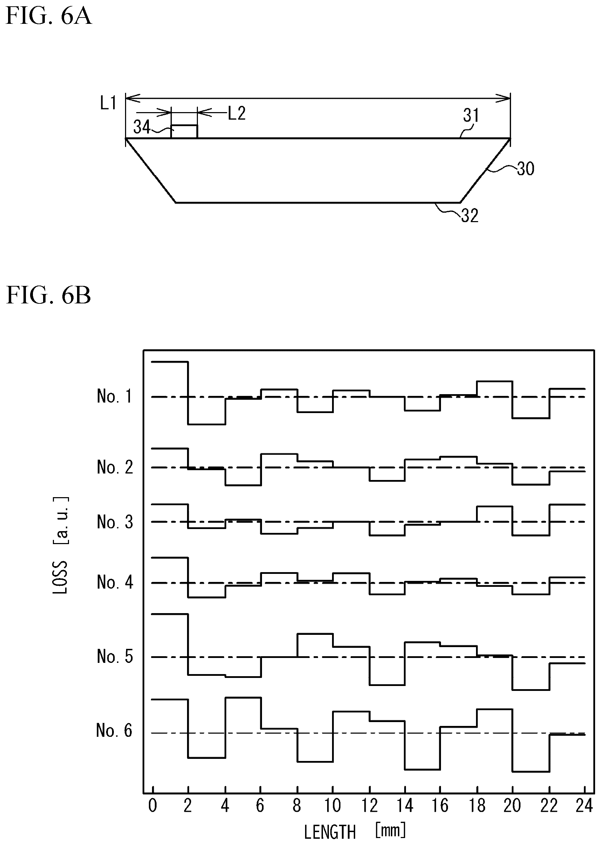

Fig. 6A illustrates a sample used in the experiment. As illustrated in Fig. 6A, a tape 34 is attached to bottom surface 31 of prism 30. The length L1 of prism 30 was 24 mm, and the length L2 of tape 34 was 2 mm. Tape 34 was attached to one end of prism 30, and an absorption by tape 34 was measured using the blood-sugar-level measuring apparatus corresponding to each example, with laser light L incident thereon. Thereafter, tape 34 was attached to a position shifted by 2 mm, and the measurement was performed again. The above procedure was repeated until the position reached the other end of prism 30. Tape 34 was a CHUKOH FLO(Trade Mark) tape formed of a fluororesin.

The wavelength of the light was 8.68 μm. The incident angles in the examples according to Embodiments 1 and 3, and the comparative example 2 were 45°, and the incident angles in the examples according to Embodiments 2, 4 and 5 were 40°. The focal lengths of lens 20 in the examples according to Embodiments 1 and 4 were 6 mm. The angles k of tip portions 22a of optical fibers 22 in the examples according to Embodiments 3 and 5 were 10°.

Fig. 6B is a diagram illustrating the measurement results of the loss. A horizontal axis represents the length of bottom surface 31 of prism 30, and a vertical axis represents the light loss normalized by an average value in each example. Nos. 1 to 5 correspond to each example according to Embodiments 1 to 5 in order. No. 6 corresponds to the comparative example 2. In each example, an alternate long and short dash line represents the average value, and a solid line represents the loss. A region from one end to the other end of prism 30 is set to 12 regions for each 1 mm, and the loss for each region is indicated by a solid line.

Variations are smaller in Nos. 1 to 5 corresponding to the examples according to Embodiments 1 to 5 than in No. 6 corresponding to the comparative example 2. Particularly in Nos. 2 to 4, the variations are small. Table 1 illustrates the coefficients of variation.

[Table 1]

|  | No. 1 | No. 2 | No. 3 | No. 4 | No. 5 | No. 6 |
|---|---|---|---|---|---|---|
| Coefficient of variation | 0.253 | 0.172 | 0.162 | 0.159 | 0.327 | 0.387 |

As illustrated in Table 1, the coefficient of variation of No. 6 corresponding to the comparative example 2 is 0.387, which is the largest among the examples. The coefficients of variation of the examples according to Embodiments 1 to 5 (Nos. 1 to 5) are smaller than that of the comparative example 2, and in particular, the coefficient of variation of the example according to Embodiment 4 is 0.159, which is the smallest in this experiment. As described above, in the examples according to Embodiments 1 to 5, incident light from the QCL on prism 30 can be made nearly uniform. Therefore, the measurement accuracy can be improved.

Reference Signs List

| 10, 12 | QCL |
|---|---|
| 20, 48 | lens |
| 22, 23 | optical fiber |
| 22a | tip portion |
| 22b | straight portion |
| 24, 25 | detector |
| 30 | prism |
| 31, 32 | bottom surface |
| 34 | tape |
| 41 | package |
| 41a | lead pin |
| 42 | TEC |
| 44 | submount |
| 46 | cap |
| 50 | lip |
| 100, 200 | blood-sugar-level measuring apparatus |

**Claims**

1. A blood-sugar-level measuring apparatus using infrared spectroscopy, comprising:

   a light source configured to emit a laser light in an infrared region;
   a lens configured to converge the laser light;
   an optical fiber in which the laser light converged by the lens enters; and
   a prism in which the laser light emitted from the optical fiber enters.

2. A blood-sugar-level measuring apparatus using infrared spectroscopy, comprising:

   a light source configured to emit a laser light in an infrared region;
   a lens configured to converge the laser light; and
   a prism in which the laser light converged by the lens enters.

3. The blood-sugar-level measuring apparatus according to claim 1 or claim 2, wherein the lens has a focal length of 2 mm or more and 20 mm or less.

4. A blood-sugar-level measuring apparatus using infrared spectroscopy, comprising:

   a light source configured to emit a laser light in an infrared region; and

a prism in which the laser light enters,

wherein the prism has a surface by which the laser light is reflected, and a lower limit of an incident angle of the laser light with respect to the surface is a critical angle plus 3°.

5. A blood-sugar-level measuring apparatus using infrared spectroscopy, comprising:

a light source configured to emit a laser light in an infrared region;

an optical fiber in which the laser light enters, and configured to have a tapered shape tapered at a tip portion thereof; and

a prism in which the laser light emitted from the tip portion enters.

6. The blood-sugar-level measuring apparatus according to any one of claims 1 to 5, wherein the light source is a quantum cascade laser.

7. A blood-sugar-level measuring method using infrared spectroscopy, the method comprising:

bringing a prism into contact with a subject,

causing a laser light in an infrared region to enter the prism through a converging lens and an optical fiber, and

detecting the laser light emitted from the prism.

8. A blood-sugar-level measuring method using infrared spectroscopy, the method comprising:

bringing a prism into contact with a subject,

causing a laser light in an infrared region to enter the prism through a converging lens, and

detecting the laser light emitted from the prism.

9. A blood-sugar-level measuring method using infrared spectroscopy, the method comprising:

bringing a prism into contact with a subject;

causing a laser light in an infrared region to enter the prism; and

detecting the laser light emitted from the prism,

wherein the prism has a surface by which the laser light is reflected, and a lower limit of an incident angle of the laser light with respect to the surface is a critical angle plus 3°.

10. A blood-sugar-level measuring method using infrared spectroscopy, the method comprising:

bringing a prism into contact with a subject;

causing a laser light in an infrared region to enter the prism through an optical fiber; and

detecting the laser light emitted from the prism,

wherein the optical fiber has a tapered shape tapered at a tip portion thereof.

FIG. 1A

FIG. 1B

FIG. 1C

FIG. 2A

FIG. 2B

FIG. 2C

FIG. 3A

41a    41    42    44    10    46    40    48

FIG. 3B

20    L    30

FIG. 4A

FIG. 4B

FIG. 5

FIG. 6A

FIG. 6B

## INTERNATIONAL SEARCH REPORT

| International application No. |
| --- |
| PCT/JP2020/000677 |

A. CLASSIFICATION OF SUBJECT MATTER
Int. Cl. G01N21/3577(2014.01)i, G01N21/552(2014.01)i, A61B5/1455(2006.01)i
FI: G01N21/552, G01N21/3577, A61B5/1455
According to International Patent Classification (IPC) or to both national classification and IPC

B. FIELDS SEARCHED

Minimum documentation searched (classification system followed by classification symbols)
Int. Cl. G01N21/00-21/01, G01N21/17-21/61, A61B5/1455

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
| --- | --- | --- |
| X | WO 2018/123169 A1 (MITSUBISHI ELECTRIC CORP.) 05 July 2018, paragraphs [0015], [0021], [0030], [0122]-[0134], fig. 13 | 2-4, 6, 8-9 |
| Y | JP 2017-140159 A (MATSUURA, Yuji) 17 August 2017, paragraphs [0014], [0015], [0017], [0020], fig. 1 | 1, 5, 7, 10 |
| Y | JP 2005-192612 A (OLYMPUS CORP.) 21 July 2005, paragraph [0039], fig. 4 | 1, 7 |
| Y | JP 2005-106592 A (AIME TECHNOLOGY CO., LTD.) 21 April 2005, paragraph [0022], fig. 5 | 1, 7 |
| Y | JP 2004-361289 A (OLYMPUS CORP.) 24 December 2004, paragraph [0039], fig. 5 | 1, 7 |

☒ Further documents are listed in the continuation of Box C.   ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 20.02.2020 | 10.03.2020 |

| Name and mailing address of the ISA/ | Authorized officer |
| --- | --- |
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/000677

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-524761 A (RIO GRANDE MEDICAL TECHNOLOGIES INC.) 19 August 2003, paragraphs [0051], [0052] | 5, 10 |
| Y | JP 2002-340796 A (SAMSUNG ELECTRONICS CO., LTD.) 27 November 2002, claims 15, 31, paragraphs [0017], [0048] | 5, 10 |

Form PCT/ISA/210 (continuation of second sheet) (January 2015)

**INTERNATIONAL SEARCH REPORT**

Information on patent family members

International application No.

PCT/JP2020/000677

| Patent Documents referred to in the Report | Publication Date | Patent Family | Publication Date |
|---|---|---|---|
| WO 2018/123169 A1 | 05.07.2018 | DE 112017006536 T<br>CN 110087544 A | |
| JP 2017-140159 A | 17.08.2017 | (Family: none) | |
| JP 2005-192612 A | 21.07.2005 | (Family: none) | |
| JP 2005-106592 A | 21.04.2005 | (Family: none) | |
| JP 2004-361289 A | 24.12.2004 | (Family: none) | |
| JP 2003-524761 A | 19.08.2003 | US 2004/0033618 A1<br>paragraphs [0101],<br>[0102]<br>WO 2000/022413 A1<br>EP 1279025 A1<br>CA 2347494 A1<br>CN 1330767 A | |
| JP 2002-340796 A | 27.11.2002 | US 2002/0169368 A1<br>claims 9-10, 31-32,<br>paragraph [0059]<br>KR 10-2002-0085625 A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019016222 A
- WO 2006011487 A
- JP 2014018478 A